# EUROPÄISCHE PATENTSCHRIFT

(11) **EP 0 591 176 B1**
(45) Veröffentlichungstag und Bekanntmachung des Hinweises auf die Patenterteilung: **06.03.1996**
(21) Anmeldenummer: 91910958.7
(22) Anmeldetag: 20.06.1991
(51) Int. Cl.: C07C 53/06, C07C 51/41

(54) **VERFAHREN ZUR HERSTELLUNG EINER ALKALI- UND/ODER ERDALKALIHALTIGEN ALUMINIUMTRIFORMIATLÖSUNG, HOCHKONZENTRIERTE ALKALI- UND/ODER ERDALKALIHALTIGE ALUMINIUMTRIFORMIATLÖSUNGEN UND IHRE VERWENDUNG**
METHOD FOR THE PREPARATION OF AN ALUMINIUM TRIFORMATE SOLUTION CONTAINING ALKALI METALS AND/OR ALKALINE-EARTH METALS, HIGHLY CONCENTRATED ALUMINIUM TRIFORMATE SOLUTIONS CONTAINING ALKALI METALS AND/OR ALKALINE-EARTH METALS, AND THE USE OF SUCH SOLUTIONS
PROCEDE DE PREPARATION DE SOLUTIONS DE TRIFORMIATE D'ALUMINIUM ALCALIFERE ET/OU CONTENANT DES BASES ALCALINO-TERREUSES, SOLUTIONS TRES CONCENTREES DE TRIFORMIATE D'ALUMINIUM ALCALIFERE ET/OU CONTENANT DES BASES ALCALINO-TERREUSES ET LEUR UTILISATION

(43) Veröffentlichungstag der Anmeldung: 13.04.1994
(73) Patentinhaber: Henkel Kommanditgesellschaft auf Aktien, D-40191 Düsseldorf (DE)
(72) Erfinder: DE RIESE-MEYER, Loert, D-4000 Düsseldorf 13 (DE); ZAUNS-HUBER, Rudolf, D-4000 Düsseldorf 13 (DE)
(86) Internationale Anmeldenummer: EP9101139
(87) Internationale Veröffentlichungsnummer: WO9300321

(56) Entgegenhaltungen:
- DE-C- 398 406
- DE-C- 873 696
- US-A- 2 289 286

## Beschreibung

Die vorliegende Erfindung betrifft ein Verfahren zur Herstellung einer alkali- und/oder erdalkalihaltigen Aluminiumtriformiatlösung durch Neutralisation einer Aluminatlauge mit einer vorgelegten ameisensäurehaltigen Lösung, die daraus resultierenden, hochkonzentrierten, alkali- und/oder erdalkalihaltigen Aluminiumtriformiatlösungen und deren Verwendung zum Imprägnieren von Textilien, in Desinfektions- und Reinigungsmitteln, als Koagulationsmittel für Lacke oder als Fixierhilfsmittel in der Lederbearbeitung.

Aus dem Stand der Technik ist bekannt, daß man wäßrige Aluminiumtriformiatlösungen entweder durch Umsetzung von frisch gefälltem Aluminiumhydroxid mit Ameisensäure oder durch die Umsetzung von Bariumformiat mit Aluminiumsulfat in Wasser gewinnen kann (siehe Ullmann, Band 3, Seite 448, 1953). Die Nachteile dieser Verfahren liegen allerdings zum einen in der für technische Zwecke störenden Alterung von Aluminiumhydroxiden sowie andererseits im Bariumformiat, daß wegen seines hohen Preises eine geringere Rentabilität besitzt. Die Umsetzungen von Ameisensäure mit anderen reaktiven Aluminiumverbindungen, wie Aluminiumhalogeniden oder Aluminiumhydroxichlorid, besitzen den Nachteil, daß sie zu einer zusätzlichen Halogenidbelastung des Produktes führen.

Aus der Lehre der deutschen Offenlegungsschrift DE-A-23 25 018 ist ein elektrochemisches Verfahren zur Herstellung von Aluminiumtriformiatlösungen bekannt, bei dem Aluminium elektrochemisch in Ameisensäure aufgelöst wird. Dieses Verfahren ist jedoch aus anlagentechnischer Sicht als sehr aufwendig zu betrachten.

Weiterhin ist bekannt, daß die Herstellung von hochkonzentrierten Aluminiumtriformiatlösungen Schwierigkeiten bereitet. So löst sich beispielsweise festes Aluminiumtriformiat in kaltem Wasser nur sehr wenig und auch in kochendem Wasser nur zu einer etwa 25 gew.-%igen wäßrigen Lösung auf (siehe Kirk-Othmar, Enzyklopädie of Chemical Technology, 3. Auflage, Band 2, Seite 204, 1978). Ein weiterer Nachteil dieser übersättigten, heißen Aluminiumtriformiatlösungen liegt darin, daß beim Abkühlen das Aluminiumtriformiat teilweise wieder auskristallisiert. Ausgehend von diesen Herstellungsschwierigkeiten von hochkonzentrierten Aluminiumtriformiatlösungen erhält man im Handel nur Aluminiumtriformiat als Feststoff oder basische Aluminiumformiate mit einem beträchtlichen Anteil an freien Hydroxylgruppen. Aus anwendungstechnischen Gründen, wie Handhabbarkeit oder Dosierung, sind jedoch hochkonzentrierte Lösungen erwünscht.

Die Aufgabe der vorliegenden Erfindung besteht nunmehr darin, ein Verfahren zur Herstellung von Aluminiumtriformiatlösungen zur Verfügung zu stellen, das auf eine korrosionsfördernde Halogenidbelastung verzichtet, von einfach zugänglichen und billigen Rohstoffen ausgeht und zu möglichst gut handhab- und dosierbaren, hochkonzentrierten Lösungen führt.

Gegenstand der vorliegenden Erfindung ist dementsprechend ein Verfahren zur Herstellung einer alkali- und/oder erdalkalihaltigen Aluminiumtriformiatlösung aus einer Aluminatlauge mit Ameisensäure, dadurch gekennzeichet, daß man eine wäßrige alkali-und/oder erdalkalihaltige Aluminatlauge mit einer vorgelegten Ameisensäure neutralisiert
wobei man die Neutralisation der alkali- und/oder erdalkalihaltigen Aluminatlauge mit Ameisensäure
i) bei einem Aluminiumgehalt von 10 bis 80 g/l bei Temperaturen von 5 bis 110 °C und
ii) bei einem Aluminiumgehalt von 80 bis 350 g/l bei Temperaturen unterhalb von 25 °C
durchführt und den ausgefallenen Feststoff unter Temperaturerhöhung in eine klare Aluminiumtriformiatlösung überführt.

Die Vorteile des erfindungsgemäßen Verfahrens liegen einerseits in der hohen Konzentration und Stabilität der erhaltenen alkali- und/oder erdalkalihaltigen Aluminiumtriformiatlösungen und andererseits in den geringen Rohstoff- bzw. Herstellkosten der Aluminatlauge, da diese beispielsweise auch bei der Herstellung von Zeolithen verwendet wird. Wenngleich nun im Sinne der Erfindung die Herstellung hochkonzentrierter Aluminiumtriformiatlösungen im Vordergrund steht, so können bei Anwendung des erfindungsgemäßen Verfahrens gewünschtenfalls auch weniger konzentrierte Aluminiumtriformiatlösungen hergestellt werden. Der Fachmann wird hierzu die Einsatzkonzentrationen der Ausgangsstoffe der angestrebten Endkonzentration der Aluminiumtriformiatlösung entsprechend anpassen und die Reaktionsbedingungen - wie nachstehend noch beschrieben - entsprechend wählen. Das erfindungsgemäße Verfahren umfaßt somit sowohl die Herstellung hochkonzentrierter als auch weniger konzentrierter Aluminiumformiatlösungen.

Im Sinne dieser Erfindung versteht man unter dem Begriff Aluminatlauge eine wäßrige Lösung eines Gemisches aus Aluminiumoxid und/oder -hydroxid und Alkali- und/oder Erdalkalioxiden und/oder -hydroxiden, vorzugsweise Natriumoxid und/oder -hydroxid, mit einem Aluminiumgehalt von 10 bis 350 g/l, vorzugsweise 70 bis 200 g/l und einem Alkali- bzw. Erdalkaligehalt, vorzugsweise Natriumgehalt, von 10 bis 350 g/l, vorzugsweise 130 bis 220 g/l.

Erfindungsgemäß können in den Aluminatlaugen prinzipiell alle denkbaren Oxide und/oder Hydroxide von Alkali- und Erdalkalimetallen enthalten sein. Besonders bevorzugt sind jedoch die Oxide und/oder Hydroxide von Natrium, Kalium, Calcium, Magnesium und/oder Barium. Gelegentlich vorhandene Eisenoxidspuren führen im allgemeinen zu einer geringfügigen Gelbfärbung in den Aluminiumtriformiatlösungen, die deren Anwendbarkeit jedoch nicht sonderlich einschränken.

In einer bevorzugten Ausführungsform dieser Erfindung wird zur Herstellung einer natriumhaltigen Aluminiumtriformiatlösung eine wäßrige Natriumaluminatlauge mit einem Natriumoxid/Aluminiumoxid-Gewichtsverhältnis von 5 : 1 bis 1 : 5, vorzugsweise von 2 : 1 bis 1 : 2, eingesetzt. In kommerziell erhältlichen Natriumaluminatlaugen beträgt das Natriumoxid/Aluminiumoxid-Gewichtsverhältnis aus Stabilitätsgründen im allgemeinen etwa 0,5 bis 1,5, da bei einem zu niedrigen Natriumhydroxidgehalt Aluminiumtrihydroxid ausfallen kann. Die wäßrige Natriumaluminatlaugen können je nach ihrer Herkunft mit verschiedenen anderen Alkali- und/oder Erdalkalimetallkationen verunreinigt sein. Auf diese Weise können beispielsweise Kalium- oder Bariumkationen bis zu einigen Gewichtsprozenten in die wäßrige Natriumaluminatlauge und folglich auch in die Aluminiumtriformiatlösung in Form ihrer Mono- bzw. Diformiate eingeschleust werden.

Im Rahmen des erfindungsgemäßen Verfahrens wird die Neutralisation der Natriumaluminatlauge bei einem Aluminiumgehalt von 10 bis 80 g/l ohne Außenkühlung unter Ausnützung der freiwerdenden Neutralisationswärme bei Temperaturen von 5 bis 110 °C, vorzugsweise 25 bis 80 °C, durchgeführt. Bei einem Aluminiumgehalt von 80 bis 350 g/l, vorzugweise 80 bis 220 g/l, muß dagegen bei Temperaturen unterhalb von 25 °C, vorzugsweise unterhalb von 15 °C, gearbeitet werden. Prinzipiell kann bei Temperaturen vom Gefrierpunkt der jeweilig eingesetzten Lösung bis unterhalb 25 °C, vorzugsweise bis unterhalb 15 °C, gearbeitet werden. Infolge des Schmelzpunktes der jeweils eingesetzten Ameisensäure, der bei 100 %iger Ameisensäure bei 8,4 °C und bei 85 %iger wäßriger Ameisensäure bei -13,8 °C liegt, wird man jedoch insbesondere bei zu hohen Aluminiumgehalten mit einer angemessenen Außenkühlung arbeiten.

Diese erfindungsgemäße Bevorzugung von niedrigeren Reaktionstemperaturen bei zunehmendem Aluminiumgehalt in der Natriumaluminatlauge läßt sich darauf zurückführen, daß die Neutralisationswärme bei zu hohem Aluminiumgehalt zu Reaktionstemperaturen über 80 °C führen kann. Bei diesen Temperaturen fällt dann ein Teil des Aluminiums in Form von Aluminiumoxid aus, so daß keine hochkonzentrierten Aluminiumtriformiatlösungen hergestellt werden können.

Im Rahmen dieser Erfindung kann als Ameisensäure eine reine 100 %ige Ameisensäure oder beliebige Abmischungen mit Wasser, vorzugsweise eine 80 bis 99 gew.-%ige wäßrige Ameisensäurelösung, eingesetzt werden.

In einer allgemeinen Ausführungsform dieser Erfindung wird die Neutralisation der alkali- und/oder erdalkalihaltigen Aluminatlauge mit der Ameisensäure in der Weise durchgeführt, daß man die Ameisensäure zur Gewährleistung eines sauren pH-Wertes vorlegt und die Aluminatlauge kontinuierlich oder diskontinuierlich zuführt. In einer bevorzugten Ausführungsform wird die Neutralisation der stark alkalischen Aluminatlauge mit der vorgelegten Ameisensäure von einem anfänglichen pH-Wert von etwa 0 bis 2 bis zu einem pH-Wert von höchstens 4,5, vorzugsweise von höchstens 4, durchgeführt. Die erfindungsgemäße Begrenzung des pH-Wertes dieser Neutralisationsreaktion läßt sich auf einfache Weise durch den Ausfall von Aluminiumtrihydroxid und basischem Aluminiumformiat bei höheren, alkalischen pH-Werten erklären. Prinzipiell kann man deswegen auch bis zu neutralen oder alkalischen pH-Werten neutralisieren, wobei man auf diese Weise jedoch Konzentrationsverluste in Kauf nehmen muß.

Im Sinne dieser Erfindung wird mit einem Aluminium/Ameisensäure-Molverhältnis von 1 : 1 bis 1 : 10, vorzugsweise 1 : 2,5 bis 1 : 3,5, gearbeitet. Um einen stöchiometrischen Umsatz zu gewährleisten wird ein Aluminium/Ameisensäure-Molverhältnis von 1 : 3 besonders bevorzugt. Bei der Angabe des Aluminium/Ameisensäure-Molverhältnis muß jedoch beachtet werden, daß auch die alkali- und/oder erdalkalihaltigen Kationen Ameisensäure verbrauchen werden. Aus diesem Grund wird man die Ameisensäure im allgemeinen im Überschuß - über die vorstehend angegebenen Molverhältnisse hinaus - einsetzen, wobei dieser Überschuß entsprechend dem Anteil an weiteren Kationen im Reaktionsgemisch, d. h. Alkalimetall - und/oder Erdalkalimetallionen, gewählt wird.

Bei der erfindungsgemäßen Neutralisationsreaktion fällt in der Regel ein gelartiger, weißer Feststoff an, der in einer nachgeschalteten Aufheizstufe wieder in Lösung gebracht werden muß. Zur erfindungsgemäßen Herstellung einer klaren, hochkonzentrierten Aluminiumtriformiatlösung wird dieser weiße Feststoff bei einer Temperatur von 25 bis 110 °C, vorzugsweise 50 bis 80 °C, bis zum Erhalt einer klaren Lösung intensiv gerührt. Im allgemeinen ist hierfür ein Zeitraum von 5 bis 60 Minuten ausreichend, um den Feststoff aufzulösen und zu einer klaren, stabilen Lösung zu gelangen.

Im Sinne dieser Erfindung können auch vor und/oder nach der Neutralisation Stabilisierungsmittel eingesetzt werden, wie vorzugsweise Ethylendiamintetraessigsäure, Sulfophthalsäure, Sulfosalicylsäure oder die wasserlöslichen Salze dieser Säuren, insbesondere deren Alkalimetallsalze, wobei die entsprechenden Natriumsalze besonders bevorzugt sind, und/oder Isopropanol, die gewünschtenfalls in Abmischung mit organischen Lösungsmitteln, wie Alkoholen oder anderen mit Wasser gut mischbaren Lösungsmitteln, und/oder Wasser eingesetzt werden können. Die jeweiligen Konzentrationen dieser Stabilisierungsmittel, die im allgemeinen im Bereich von 0,1 bis 10 Gew.-%, vorzugsweise 0,5 bis 2 Gew.-%, liegen, muß der Fachmann je nach den Gegebenheiten variieren. So kann beispielsweise zur Erhöhung der Lagerstabilität der Aluminiumtriformiatlösungen - bei gegebenenfalls niederen Temperaturen - ein Zusatz an Stabilisierungsmitteln durchaus in Frage kommen. Hierbei richtet sich die zugesetzte Menge an Stabilisierungsmittel nach der Konzentration der jeweiligen Aluminiumtriformiatlösung, d. h. je höher die Konzentration dieser Lösung ist, desto höher wird auch die Einsatzmenge an Stabilisierungsmittel gewählt

Ein weiterer Gegenstand dieser Erfindung sind die nach dem oben beschriebenen Verfahren hergestellten hochkonzentrierten, alkali- und/oder erdalkalihaltigen Aluminiumtriformiatlösungen mit einem Alkali- und/oder Erdalkaliformiat-Gehalt von 1 bis 50 Gew.-%, vorzugsweise 5 bis 30 Gew.-%, und einem Aluminiumtriformiat-Gehalt von 25 bis 50 Gew.-%, vorzugsweise 30 bis 40 Gew.-%. Diese hochkonzentrierten, alkali- und/oder erdalkalihaltigen Aluminiumtriformiatlösungen erhält man insbesondere dann, wenn man mit einem hohen Aluminiumgehalt bei möglichst tiefen Temperaturen arbeitet.

Als mögliche Einsatzbereiche für diese hochkonzentrierten Aluminiumtriformiatlösungen kommen insbesondere die aus dem Stand der Technik bekannten Bereiche in Frage, in denen ein störender Gehalt von Alkali- und/oder Erdalkaliformiaten keine Auswirkungen hat.

Ein weiterer Gegenstand dieser Erfindung ist die Verwendung der erfindungsgemäßen hochkonzentrierten, alkali- und/oder erdalkalihaltigen Aluminiumtriformiatlösungen beim Imprägnieren von Textilien, in Desinfektions- und Reinigungsmitteln, als Koagulationsmittel für Lacke oder als Fixierhilfsmittel in der Lederbearbeitung. Weiterhin können die erfindungsgemäßen Aluminiumtriformiatlösung beim Leimen von Papier, bei der Silierung von Grünfutter oder als Beize in der Textil- und Rauchwarenindustrie Verwendung finden.

### Beispiele

### Beispiel 1

Zu 1030 g Ameisensäure (100 %) wurden bei Raumtemperatur 2000 g einer wäßrigen Natriumaluminatlauge, die 14,3 Gew.-% Natriumoxid und 11,6 Gew.-% Aluminiumoxid enthielt, innerhalb von 20 Minuten zugetropft. Dabei fand ein Temperaturanstieg bis 80 °C und eine Niederschlagsbildung statt. Anschließend wurde die Mischung bis zum Erhalt einer klaren Lösung für ca. 15 bis 30 Minuten unter Rückfluß erwärmt. Die gelbe Lösung enthielt bei einem pH-Wert von 3,9 ca. 24,3 Gew.-% Aluminiumtriformiat und 20,7 Gew.-% Natriumformiat.

### Beispiel 2

Zu 1210 g einer 85 %igen wäßrigen Ameisensäure wurden bei Raumtemperatur 2000 g einer wäßrigen Natriumaluminatlauge, die 14,3 Gew.-% Natriumoxid und 11,6 Gew.-% Aluminiumoxid enthielt, innerhalb von 20 Minuten zugetropft. Dabei fand ein Temperaturanstieg bis ca. 80 °C und eine Niederschlagsbildung statt. Anschließend wurde die Mischung bis zum Erhalt einer klaren Lösung für ca. 15 bis 30 Minuten unter Rückfluß erwärmt. Die gelbe Lösung enthielt bei einem pH-Wert von 3,9 ca. 23 Gew.-% Aluminiumtriformiat und 19,5 Gew.-% Natriumformiat.

### Beispiel 3

Zu 2260 g einer 85 %igen wäßrigen Ameisensäure wurden unter Außenkühlung mit kaltem Wasser 2000 g einer wäßrigen DYNAFLOC L-Lösung (Dynamit Nobel, 19 Gew.-% Natriumoxid und 25 Gew.-% Aluminiumoxid) unter starkem Rühren zugetropft. Die Tropfgeschwindigkeit richtete sich nach der Temperatur der Reaktionslösung, die 15 °C nicht überschreiten sollte. Nach etwa 45 Minuten wurde die Mischung bei 60 °C bis zum Erhalt einer klaren Lösung gerührt. Die farblose Lösung enthielt bei einem pH-Wert von 3,5 etwa 37,3 Gew.-% Aluminiumtriformiat und 19,6 Natriumformiat.

### Beispiel 4

Zu 1920 g Ameisensäure (100 %) wurden unter Außenkühlung mit kaltem Wasser und starkem Rühren 2000 g einer wäßrigen DYNAFLOC L-Lösung (Dynamit Nobel; 19 Gew.-% Na₂O/25 Gew.-% Al₂O₃) zugetropft. Die Tropfgeschwindigkeit richtete sich nach der Temperatur der Reaktionslösung, die 15 °C nicht übersteigen sollte (Dauer ca. 45 Minuten). Anschließend wurde bei 60 °C bis zum Erhalt einer klaren Lösung (ca. 10 Minuten) gerührt. Die farblose Lösung enthielt bei einem pH-Wert von 3,7 40,5 Gew.-% Aluminiumtriformiat und 21,3 Gew.-% Natriumformiat.

### Beispiel 5

Zu 2260 g einer 85 %igen wäßrigen Ameisensäure wurden unter Außenkühlung mit kaltem Wasser und starkem Rühren 2000 g einer wäßrigen Aluminatlauge (15,3 Gew.-% Na₂O/20,2 Gew.-% Al₂O₃) zugetropft. Die Tropfgeschwindigkeit richtete sich nach der Temperatur der Reaktionslösung, die 25 °C nicht übersteigen sollte (Dauer ca. 20 Minuten). Anschließend wurde bei 60 °C bis zum Erhalt einer klaren Lösung (ca. 10 Minuten) gerührt. Die farblose Lösung enthielt bei einem pH-Wert von 3,7 30,1 Gew.-% Aluminiumtriformiat und 15,8 Gew.-% Natriumformiat.

## Patentansprüche

1. Verfahren zur Herstellung einer alkali- und/oder erdalkalihaltigen Aluminiumtriformiatlösung aus einer Aluminatlauge mit Ameisensäure, dadurch gekennzeichnet, daß man eine wäßrige alkali- und/oder erdalkalihaltige Aluminatlauge mit einer vorgelegten Ameisensäure neutralisiert, wobei man die Neutralisation der alkali- und/oder erdalkalihaltigen Aluminatlauge mit Ameisensäure
i) bei einem Aluminiumgehalt von 10 bis 80 g/l bei Temperaturen von 5 bis 110 °C und
ii) bei einem Aluminiumgehalt von 80 bis 350 g/l bei Temperaturen unterhalb von 25 °C
durchführt und den ausgefallenen Feststoff unter Temperaturerhöhung in eine klare Aluminiumtriformiatlösung überführt.

2. Verfahren nach Anspruch 1, dadurch gekennzeichnet, daß man als alkali- und/oder erdalkalihaltige Aluminatlauge eine wäßrige Lösung eines Gemisches aus Aluminiumoxid und/oder -hydroxid und Alkali- und/oder Erdalkalioxiden und/oder -hydroxiden mit einem Aluminiumgehalt von 10 bis 350 g/l, vorzugsweise 70 bis 200 g/l, und einem Alkali- und/oder Erdalkaligehalt, vorzugsweise Natriumgehalt, von 10 bis 350 g/l, vorzugsweise 130 bis 220 g/l, einsetzt.

3. Verfahren nach den Ansprüchen 1 und 2, dadurch gekennzeichnet, daß man eine wäßrige Natriumaluminatlauge mit einem Natriumoxid/Aluminiumoxid-Gewichtsverhältnis von 5 : 1 bis 1 : 5, vorzugsweise von 2 : 1 bis 1 : 2, einsetzt.

4. Verfahren nach den Ansprüchen 1 bis 3, dadurch gekennzeichnet, daß man die Neutralisation der alkali- und/oder erdalkalihaltigen Aluminatlauge mit der vorgelegten Ameisensäure bis zu einem pH-Wert von höchstens 4,5, vorzugsweise von höchstens 4, durchführt.

5. Verfahren nach den Ansprüchen 1 bis 4, dadurch gekennzeichnet, daß man die Neutralisation der Aluminatlauge mit einem Aluminium/Ameisensäure-Molverhältnis von 1 : 1 bis 1 : 10, vorzugsweise 1 : 2,5 bis 1 : 3,5, durchführt.

6. Verfahren nach den Ansprüchen 1 bis 5, dadurch gekennzeichnet, daß man den nach der Neutralisation ausgefallenen Feststoff bei einer Temperatur von 25 bis 110 °C, vorzugsweise 50 bis 80 °C, unter Rühren in eine klare Lösung überführt.

7. Verfahren nach den Ansprüchen 1 bis 6, dadurch gekennzeichnet, daß man vor und/oder nach der Neutralisationsreaktion Stabilisierungsmittel, vorzugsweise Ethylendiamintetraessigsäure, Sulfophthalsäure, Sulfosalicylsäure oder die wasserlöslichen Salze dieser Säuren und/oder Isopropanol, gewünschtenfalls in Abmischung mit organischen Lösungsmitteln und/oder Wasser, einsetzt.

8. Hochkonzentrierte, alkali- und/oder erdalkalihaltige wäßrige Aluminiumtriformiatlösungen, dadurch gekennzeichnet, daß sie einen Alkali- und/oder Erdalkaliformiat-Gehalt von 1 bis 50 Gew.-%, vorzugsweise 5 bis 30 Gew.-%, und einen Aluminiumtriformiat-Gehalt von 25 bis 50 - Gew.-%, vorzugsweise 30 bis 40 Gew.-%, aufweisen.

9. Verwendung der alkali- und/oder erdalkalihaltigen Aluminiumtriformiatlösungen nach den Ansprüchen 1 bis 8 zum Imprägnieren von Textilien, in Desinfektions- und Reinigungsmitteln, als Koagulationsmittel für Lacke oder als Fixierhilfsmittel in der Lederbearbeitung.

## Claims

1. A process for the production of an aluminium triformate solution containing alkali metal and/or alkaline earth metal from an aluminate liquor using formic acid, characterized in that an aqueous aluminate liquor containing alkali metal and/or alkaline earth metal is neutralized with a formic acid introduced beforehand, the neutralization of the aluminate liquor containing alkali metal and/or alkaline earth metal with formic acid being carried out
i) at an aluminium content of 10 to 80 g/l at temperatures of 5 to 110°C and
ii) at an aluminium content of 80 to 350 g/l at temperatures below 25°C,
and the solid precipitated is converted by heating into a clear aluminium triformate solution.

2. A process as claimed in claim 1, characterized in that an aqueous solution of a mixture of aluminium oxide and/or hydroxide and alkali metal and/or alkaline earth metal oxides and/or hydroxides with an aluminium content of 10 to 350 g/l and preferably 70 to 200 g/l and an alkali metal and/or alkaline earth metal content, preferably a sodium content, of 10 to 350 g/l and preferably 130 to 220 g/l is used as the aluminate liquor containing alkali metal and/or alkaline earth metal.

3. A process as claimed in claims 1 and 2, characterized in that an aqueous sodium aluminate liquor with a ratio by weight of sodium oxide to aluminium oxide of 5:1 to 1:5 and preferably 2:1 to 1:2 is used.

4. A process as claimed in claims 1 to 3, characterized in that the neutralization of the aluminate liquor containing alkali metal and/or alkaline earth metal with the formic acid introduced beforehand is carried out to a pH value of at most 4.5 and preferably to a pH value of at most 4.

5. A process as claimed in claims 1 to 4, characterized in that the neutralization of the aluminate liquor is carried out with a molar ratio of aluminium to formic acid of 1:1 to 1:10 and preferably 1:2.5 to 1:3.5.

6. A process as claimed in claims 1 to 5, characterized in that the solid precipitated after neutralization is converted into a clear solution with stirring at a temperature of 25 to 110°C and preferably at a temperature of 50 to 80°C.

7. A process as claimed in claims 1 to 6, characterized in that stabilizers, preferably ethylenediamine tetraacetic acid, sulfophthalic acid, sulfosalicylic acid or the water-soluble salts of these acids and/or isopropanol, if desired in admixture with organic solvents and/or water, are used before and/or after the neutralization reaction.

8. Highly concentrated aluminium triformate solutions containing alkali metal and/or alkaline earth metal, characterized in that they have an alkali metal and/or alkaline earth metal formate content of 1 to 50% by weight and preferably 5 to 30% by weight and an aluminium triformate content of 25 to 50% by weight and preferably 30 to 40% by weight.

9. The use of the aluminium triformate solutions containing alkali metal and/or alkaline earth metal claimed in claims 1 to 8 for impregnating textiles, in disinfectants and cleaning products, as coagulants for lacquers or for as fixing agents in the processing of leather.

## Revendications

1. Procédé de préparation d'une solution de triformiate d'aluminium contenant des métaux alcalins et/ou alcalino-terreux à partir d'une lessive d'aluminate, avec de l'acide formique, caractérisé en ce que l'on neutralise une lessive d'aluminate aqueuse contenant des métaux alcalins et/ou alcalino-terreux avec de l'acide formique introduit, procédé dans lequel on effectue la neutralisation de la lessive d'aluminate contenant des métaux alcalins et/ou alcalino-terreux avec de l'acide formique
i) pour une teneur en aluminium de 10 à 80 g/l, à des températures de 5 à 110°C, et
ii) pour une teneur en aluminium de 80 à 350 g/l, à des températures en dessous de 25°C,
et dans lequel on convertit la matière solide qui a précipité par élévation de la température en une solution claire de triformiate d'aluminium.

2. Procédé selon la revendication 1, caractérisé en ce que l'on met en oeuvre comme lessive d'aluminate contenant des métaux alcalins et/ou alcalino-terreux, une solution aqueuse d'un mélange d'oxyde et/ou d'hydroxyde d'aluminium et d'oxydes et/ou d'hydroxydes de métaux alcalins et/ou de métaux alcalino-terreux, avec une teneur en aluminium de 10 à 350 g/l, de préférence de 70 à 200 g/l et une teneur en métaux alcalins et/ou alcalino-terreux, de préférence une teneur en sodium, de 10 à 350 g/l, de préférence de 130 à 220 g/l.

3. Procédé selon les revendications 1 et 2, caractérisé en ce que l'on met en oeuvre une lessive d'aluminate de sodium aqueuse, avec un rapport pondéral oxyde de sodium/oxyde d'aluminium de 5:1 à 1:5, de préférence de 2:1 à 1:2.

4. Procédé selon les revendications 1 à 3, caractérisé en ce que l'on effectue la neutralisation de la lessive d'aluminate contenant des métaux alcalins et/ou alcalino-terreux avec de l'acide formique introduit jusqu'à une valeur de pH d'au maximum 4,5, de préférence d'au maximum 4.

5. Procédé selon les revendications 1 à 4, caractérisé en ce que l'on effectue la neutralisation de la lessive d'aluminate avec un rapport molaire aluminium/acide formique allant de 1:1 à 1:10, de préférence de 1:2,5 à 1:3,5.

6. Procédé selon les revendications 1 à 5, caractérisé en ce que l'on transforme la matière solide qui a précipité après la neutralisation en une solution claire à une température de 25 à 110°C, de préférence 50 à 80°C sous agitation.

7. Procédé selon les revendications 1 à 6, caractérisé en ce que l'on met en oeuvre avant et/ou après la réaction de neutralisation, un agent stabilisant, de préférence l'acide éthylènediaminetétraacétique, l'acide sulfophtalique, l'acide sulfosalicylique, ou les sels solubles dans l'eau de ces acides et/ou de l'isopropanol, si désiré en mélange avec des solvants organiques et/ou de l'eau.

8. Solutions très concentrées aqueuses de triformiate d'aluminium contenant des métaux alcalins et/ou alcalino-terreux, caractérisées en ce qu'elles possèdent une teneur en formiates alcalins et/ou alcalino-terreux allant de 1 à 50 % en poids, de préférence de 5 à 30 % en poids, et une teneur en triformiate d'aluminium allant de 25 à 50 % en poids, de préférence de 30 à 40 % en poids.

9. Utilisation des solutions de triformiate d'aluminium contenant des métaux alcalins et/ou alcalino-terreux selon les revendications 1 à 8, pour l'imprégnation des textiles, dans les agents désinfectants et les agents de nettoyage, comme agent de coagulation pour des laques ou comme adjuvants de fixation dans le traitement du cuir.
